# EUROPEAN PATENT APPLICATION

(11) **EP 1 038 961 A1**
(43) Date of publication of application: **27.09.2000**
(21) Application number: 99200773.2
(22) Date of filing: 16.03.1999
(51) Int. Cl.: C12N 15/40, C12N 15/82, A01H 5/00

(54) **Method for inducing viral resistance into a plant**

(71) Applicant: CENTRE NATIONAL DE LA RECHERCHE SCIENTIFIQUE, 75794 Paris Cédex 16 (FR)
(72) Inventor: Jonard, Gérard, 67084 Strasbourg Cédex (FR); Lauber, Emmanuelle, 67000 Strasbourg (FR); Guilley, Hubert, 67370 Berstett (FR); Richards, Kenneth, 67570 Pfulgriesheim (FR)
(74) Representative: Van Malderen, Joelle

(57) **Abstract**

The present invention concerns a method for inducing resistance to a virus comprising a TGB2 sequence into a cell plant or a plant, comprising the following steps :
- preparing a nucleotide construct comprising a nucleotide sequence corresponding to at least 70% of the nucleotide sequence of TGB2 of said virus or its complementary cDNA, being operably linked to one or more regulatory sequence(s) active in a plant,
- transforming a plant cell with the nucleotide construct, and possibly
- regenerating a transgenic plant from the transformed plant cell.

The present invention is also related to the plant obtained.

## Description

### Field of the invention

The present invention is related to a method for inducing viral resistance into a cell and a plant, especially BNYW-resistance into a sugar beet cell and plant.

### Background of the invention and state of the art

The widespread viral disease of the sugar beet plant (*Beta vulgaris*) called Rhizomania is caused by a benyvirus, the beet necrotic yellow vein virus (BNYVV) (23, 24) which is transmitted to the root of the beet by the soilborne fungus *Polymyxa betae* (25).

The disease significantly affects acreages where the sugar beet plant is grown for industrial use in Europe, USA and Japan and is still in extension in several places in Western Europe (26, 27). As there exists no practical method to effectively control the spread of the virus at a large scale by chemical or physical means (28), neither in the plants nor in the soil, the main focus has been to identify natural sources of resistance within the sugar beet germplasm and to develop by breeding, varieties of sugar beet plants expressing the resistance genes. A variety of such tolerance genes to the virus have been identified and, some have been successfully used in the breeding of commercial sugar beet varieties (29, 30, 31).

Only the use of BNYVV-resistant or tolerant varieties will enable farmers to grow sugar beet plants in BNYVV-infected areas where the sugar beet plant is an essential component of the crop rotation and contributes significantly to the grower's income.

A number of detailed studies have shown that a difference in susceptibility to the BNYVV-infection among sugar beet genotypes or varieties, generally reflect difference in the diffusion or translocation of the virus in the root tissues (32).

However, there are still few reports which indicate clearly that the tolerance genes, even from differing sources of sugar beet germplasm or wild relatives germplasm (33), would provide distinct mechanisms of resistance. Such a situation would represent a more manageable situation to design long lasting BNYW-resistance strategies.

Since 1986, a number of reports and publications have described the use of isolated viral gene sequences expressed in plants to confer a high level of tolerance against the virus or even to confer a broad spectrum type of resistance against a number of related viruses (34, 35, 36). One of the most documented viral resistance strategy based on genetic engineering, in many cultivated species such as potato, squash, cucumber or tomato, is the use of the viral gene sequence which under the control of plant regulatory elements, encodes the coat-protein of the target virus (37).

However, for coat-protein mediated resistance, the expression of a certain level of resistance in the transgenic plant might be attributed to different mechanisms such as RNA co-suppression and not necessarily to the production of the protein sequence.

In general, the virus sequence will be transferred in an appropriate cell or tissue culture of the plant species using an *Agrobacterium* mediated transformation system or a direct gene transfer method according to the constraints of the tissue culture or cell culture method which can be successfully applied in a given species. A whole plant will be regenerated and the expression of the transgene will be characterized.

Though sugar beet is known as a recalcitrant species in cell culture, limiting the extent of practical genetic engineering applications in that species, there are number of isolated reports of successful transformation and regeneration of whole plants (38). A few examples of engineering tolerance to the BNYVV by transforming and expressing the BNYVV coat-protein sequence in the sugar beet genome have also been published (39, W091/13159) though they rarely report data on whole functional transgenic sugar beet plants (40). In particular, reports show limited data on the level of resistance observed in infected conditions with transgenic sugar beet plants transformed with a gene encoding a BNYVV coat-protein sequence (41, 42).

A complete technology package including a sugar beet transformation method and the use of the expression of the BNYVV coat-protein sequence as resistance source in the transgenic sugar beet plant obtained by said transformation method has been described in the Patent Application W091/13159.

Based on the information published, it can not be concluded that the coat-protein mediated resistance mechanism provides any potential for conferring to the sugar beet plant a total immunity to the BNYVV-infection by inhibiting completely the virus multiplication and diffusion mechanisms. To identify a resistance mechanism which significantly blocks the spread of the virus at the early stage of the infection process would be a major step toward successfully developing such a transgenic resistance. In addition, such resistance would diversify the mechanisms of resistance available.

Because the disease is shown to expand in many countries or areas, at a speed depending upon the combination of numerous local environmental and agricultural factors, there is a strong interest diversifying genetic resistance mechanisms which may, alone or in combination, confer a stable and long lasting resistance strategy in the current and future varieties of sugar beet plants which are grown for industrial use.

The genome of beet necrotic yellow vein benyvirus (BNYVV) consists of five plus-sense RNAs, two of which (RNAs 1 and 2) encode functions essential for infection of all plants while the other three (RNAs 3, 4 and 5) are implicated in vector-mediated infections of host plants (*Beta macrocarpa, Beta vulgaris, Spinacear oleracea, Chenopodium quinoa,* etc.) roots (1). Cell-to-cell movement of BNYVV is governed by a set of three successive, slightly overlapping viral genes on RNA 2 known as the triple gene block (TGB) (2), which encode the viral proteins P42, P13 and P15 (gene products are designated by their calculated Mᵣ in kilodalton (3).

In the following description, the TGB genes and the corresponding proteins will be identified by the following terms: TGB1, TGB2, TGB3 or by their encoded viral protein number P42, P13 and P15. TGB counterparts are present in other plant viruses and the characteristics of their TGB have allowed the classification of said viruses in two groups: the viruses of group I which include hordéiviruses, benyviruses, pecluviruses and pomoviruses and the viruses of group II represented by potexviruses and carlaviruses (4, 5, 6, 44).

For the viruses of group II, capsid protein is also involved in the cell-to-cell movement of viruses.

The development of a resistance to viral infections into a plant by blocking the cell-to-cell movement has been described for the potato viruses X (PVX) (45) and for the white clover mosaic virus (WC1MV) (46) in *Nicotiana benthamiana.* These two viruses belong to the above-described group II. In both cases, various amino acids were replaced by Alanine in the hydrophilic part of the TGB sequence downstream of the N-terminal hydrophobic domain of said amino acid sequence. However, it was not possible with said mutants to obtain total resistance, especially when a virus challenger concentration is increasing into the plant.

### Aims of the invention

The present invention aims to provide a new method for introducing various viral resistances into a cell and a plant and the viral resistant cell and plant obtained.

A main aim of the invention is to provide a new method for introducing BNYVV resistance into a cell and a plant and the BNYVV-resistant cell and plant, in particular a sugar beet cell and plant *(Beta vulgaris* *ssp*.) , obtained.

### Summary of the invention

The present invention provides the use of an alternative sequence of plant virus, especially the BNYVV, to obtain a high degree of tolerance to the viral infection, in particular to ensure a rapid and total blocking of virus multiplication and diffusion mechanisms in a plant, especially in the sugar beet plant (*Beta vulgaris),* including fodder beet, Swiss chard and table beet, which may also be subject to this viral infection. Expression of the resistance will be obtained in transgenic cell and plant, especially sugar beet cells and plants produced by the transformation method subject to the Patent Application W095/10178 or by other transformation methods based on *Agrobacterium tumefaciens* or direct gene transfer. Because of its high efficiency, the transformation method as described in W095/10178 enables the production of large numbers of transformed plants, especially sugar beet plants, and will be preferred to develop transgenic plants which may be analysed and characterized for their level of viral resistance, especially BNYVV Resistance, including their field evaluation.

In the table 1 are represented viruses having a TGB2 sequence, the molecular weight of TGB2 of said viruses, their host and references.

**Table 1**

| **Virus** | **Size of TGB2 (kDa)** | **Host** | **Reference** |
|---|---|---|---|
| ***GROUP I*** | | | |
| Beet necrotic yellow vein virus | 13 | beet | Bouzouba et al., *J. Gen. Virol.* 67, 1689-1700 (1986) |
| Barley stripe mosaic virus | 14 | barley | Gustafson et al., *Nucl. Acids Res.* 14, 3895-3909 (1986) |
| Potato mop top virus | 13 | potato | Scott et al., *J. Gen. Virol.* 75, 3561-3568 (1994) |
| Peanut clump virus | 14 | peanut | Herzog et al., *J. Gen. Virol.* 75, 3147-3155 (1994) |
| Beet soil-borne virus | 13 | sugar beet | Koenig et al., *Virology* 216, 202-207 (1996) |

| ***GROUP II*** | | | |
|---|---|---|---|
| Apple stem pitting virus | 13 | apple | Jelkman, *J. Gen. Virol.* 75, 1535-1542 (1994) |
| Blueberry scorch virus | 12 | blueberry | Cavileer et al., *J. Gen. Virol.* 75, 711-720 (1994) |
| Potato virus M | 12 | potato | Zavriev et al., *J. Gen. Virol.* 72, 9-14 (1991) |
| White clover mosaic virus | 13 | clover | Forster et al., *Nucl. Acids Res.* 16, 291-303 (1988) |
| *Cymbidium* mosaic virus | 14 | orchid | Neo et al., *Plant Mol. Biol.* 18, 1027-1029 (1992) |

The Inventors propose herewith a new method for providing resistance to plant viruses into a plant by blocking virus multiplication and diffusion mechanisms into said plant, especially into its root tissue. In order to demonstrate said resistance, the Inventors describe hereafter the effect of the overexpression of TGB2 sequence alone or in combination upon BNYVV multiplication and diffusion mechanism in plants of *C. quinoa* which are also the hosts of the BNYVV virus and which could be more easily manipulated by the man skilled in the art.

It is known that BNYVV does not require synthesis of viral coat protein for production of local lesions on leaves of hosts such as *Chenopodium quinoa (7),* indicating that virion formation is not required for cell-to-cell movement.

However, the manner in which the TGB components assist in the movement process is not understood although computer-assisted sequence comparisons have detected characteristic conserved sequences which may provide clues to their function. Thus, the 5'-proximal TGB protein (TGB1) invariably contains a series of sequence motifs characteristic of an ATP/GTP-binding helicase while the second protein (TGB2) always has two potentially membrane-spanning hydrophobic domains separated by a hydrophilic sequence which contains a highly conserved peptide motif of unknown significance (6).

So far, no example has been reported of a virus of group I in which the three TGB members are arranged differently on the same RNA or are parcelled out to different genome RNAs, suggesting that their association in a particular order might be important in regulating their function.

The present invention concerns a method for inducing viral resistance to a virus of group I comprising the triple gene block (TGB2). Said viruses of group I comprise hordéiviruses, benyviruses, pecluviruses and pomoviruses, preferably viruses selected from the group consisting of the beet necrotic yellow vein virus, the barley stripe mosaic virus, the potato mop top virus, the peanut clump virus and the beet soil-borne virus; said method comprises the following steps:
- preparing a nucleotide construct comprising a nucleotide sequence corresponding to at least 70% of the wild-type nucleotide sequence of TGB2 of said group I virus or its corresponding cDNA, being operably linked to one or more regulatory sequence(s) active in a plant,
- transforming a plant cell with the nucleotide construct, and possibly
- regenerating the transgenic plant from the transformed plant cell.

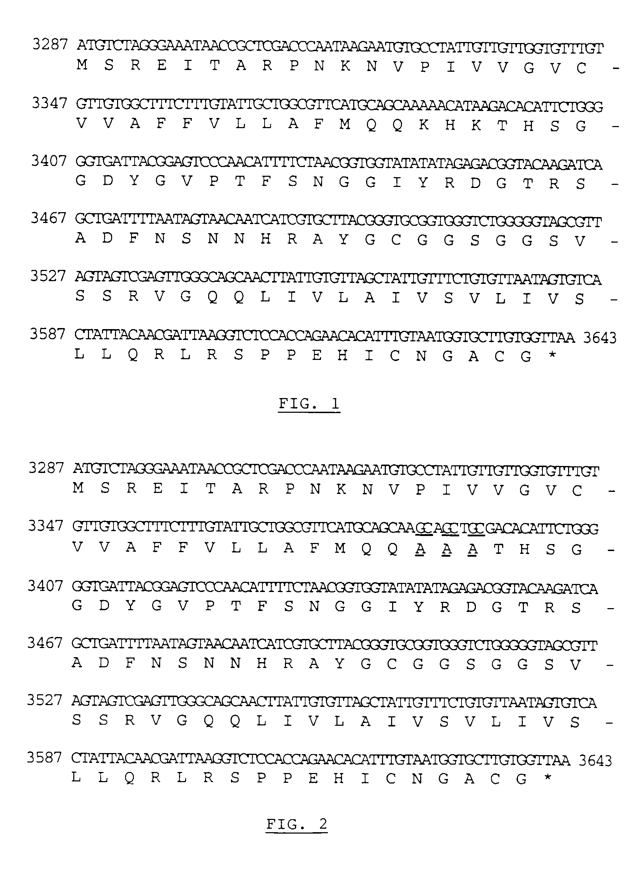

Advantageously, the nucleotide sequence corresponding to at least 70% of the wild-type nucleotide sequence of TGB2 or its corresponding cDNA comprise the substitution of at least one amino acid into another different amino acid in the TGB2 wild-type sequence SEQ ID NO. 1 (Fig. 1). Preferably, the substitution of at least one amino acid into another different amino acid is made in regions rich in hydrophilic amino acids usually present at the surface of the corresponding protein in its native configuration. Preferably, a modification is made in the hydrophilic region of the wild-type sequence downstream the N-terminal hydrophobic domain and just upstream the conserved central domain.

According to a preferred embodiment of the present invention, said amino acids are each substituted by the amino acid Alanine.

Preferably, the plant or plant cell is a plant or plant cell which may be infected by the above-described virus and is preferably selected from the group consisting of potato, barley, peanut and sugar beet.

The present invention concerns also the obtained plant cell and transgenic (or transformed) plant (made of said plant cells) resistant to said viruses and comprising said nucleotide construct.

The Inventors have also discovered unexpectedly that it is possible to induce BNYVV-resistance into a plant by a method which comprises the following steps:
- preparing a nucleotide construct comprising a nucleotide sequence corresponding to at least 70%, preferably at least 80%, more preferably at least 90%, of the wild-type nucleotide sequence comprised between the nucleotides 3287 and 3643 of the 5' strand of the genomic or subgenomic wild-type RNA 2 of the BNYVV or its corresponding cDNA, being operably linked to one or more regulatory sequence(s) active in a plant,
- transforming a plant cell with said construct, and possibly
- regenerating a transgenic plant from the transformed plant cell.

The nucleotide sequence comprised between the nucleotides 3287 and 3643 of the 5' strand of the genomic or subgenomic RNA 2 encoding the P13 protein is described in the Fig. 1 (SEQ ID NO. 1). A preferred mutated nucleotide sequence and its corresponding mutated amino acid sequence are described in the following specification as SEQ ID NO. 3 (Fig. 2).

Another aspect of the present invention concerns a plant cell and a transgenic plant (made of said plant cells) resistant to BNYVV and comprising a nucleotide construct having a nucleotide sequence corresponding to at least 70%, preferably at least 80%, more preferably at least 90%, of the nucleotide sequence comprised between the nucleotides 3287 and 3643 of the 5' strand of the genomic or subgenomic wild-type RNA 2 of BNYVV or its corresponding cDNA, being operably linked to one or more regulatory sequence(s) active in the plant.

Preferably, said plant cell or transgenic plant (made of said plant cells) resistant to BNYW is obtained by the method according to the invention.

The variants of the wild-type nucleotide sequence (SEQ ID NO. 1) comprise insertion, substitution or deletion of nucleotides encoding the same or different amino acid(s) (see Fig. 2). Therefore, the present invention concerns also said variants of the nucleotide sequence of SEQ ID NO. 1, for example SEQ ID NO. 3, which present at least 70%, preferably at least 80%, more preferably at least 90%, homology with said nucleotide sequence and which are preferably able to hybridise to said nucleotide sequence in stringent or non-stringent conditions as described by Sambrook et al., §§ 9.47-9.51 in *Molecular Cloning : A* Laboratory *Manual,* Cold Spring Harbor, Laboratory Press, Cold Spring Harbor, New York (1989).

A nucleotide sequence corresponding to at least 70%, preferably at least 80%, more preferably at least 90%, of the nucleotide sequence comprised between the nucleotides 3287 and 3643 of the 5' strand of the genomic or subgenomic wild-type RNA 2 of BNYW or its corresponding cDNA, is preferably a sequence comprising a substitution of at least one amino acid into another different amino acid in the wild-type RNA2 sequence of the BNYVV or its corresponding cDNA. Preferably said substitution is made in regions in which hydrophilic amino acids are usually present at the surface of the protein in its native configuration (47) as described in Fig. 2 (A = substitution by Alanine). Preferably, said substitution of one or more amino acids is a mutation which allows the substitution of one or more amino acids into one or more Alanine amino acids.

According to a preferred embodiment of the present invention, said nucleotide sequence is SEQ ID NO. 3.

Preferably, said sequences are also able to induce BNYVV resistance into a plant.

The terms "induce a viral resistance into a plant" mean inducing a possible reduction or a significant delay into the appearance of infection symptoms, virus multiplication or its diffusion mechanisms into the plant, especially in the root tissues.

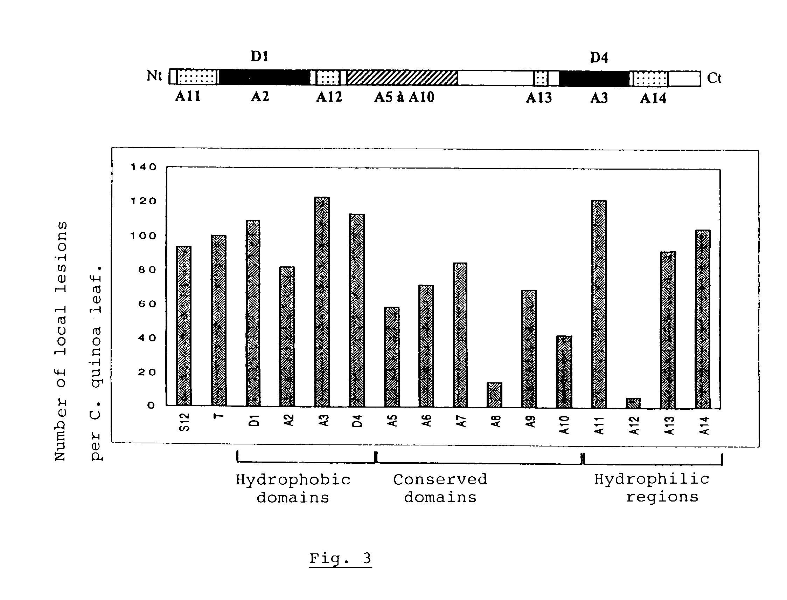

In Fig. 3 are represented results showing the capacity of a plant coinoculated with virus containing a replicon construct with the nucleotide sequence according to the invention, especially the sequence SEQ ID NO. 3, to inhibit the movement by BNYVV in *C. Quinoa.* The infectious factor of BNYVV is shown by the appearance of local lesions of leaves of said plant after co-inoculation of wild-type virus S12. Fig. 3 presents the number of local lesions upon leaves of a plant by a BNYVV S12 isolate (comprising RNA1 and RNA2) when co-inoculated with various replicons incorporating either mutated sequences including SEQ ID NO. 3 identified in Fig. 2 or a wild-type nucleotide sequence (T).

Eight days after said inoculation, the local lesions are identified. The results of three experiments show that the decreasing of said effect is mostly observed with the co-inoculation of the mutated sequence SEQ ID NO. 3 (up to 100% inhibition). This effect is not due to a possible blocking effect upon RNA1 and RNA2 replication, but the replicons according to the invention allow a blocking of the biochemical mechanisms involved in cell-to-cell movements by the infectious virus.

The regulatory sequence(s) of the nucleotide sequence according to the invention are promoter sequence(s) and terminator sequence(s) active into a plant.

The nucleotide construct may also include a selectable marker gene, which could be used to identify the transformed cell or plant and express the nucleotide construct according to the invention.

Preferably, the cell is a stomatal cell and the plant is a sugar beet (*Beta vulgaris ssp*.) made of said cells.

According to the invention, the promoter sequence is a constitutive or foreigner promoter sequence. Examples are 35S Cauliflower Mosaic Virus promoter sequence, polyubiquitin *Arabidopsis thaliana* promoter (43), a promoter which is mainly active in root tissues such as the par promoter of the haemoglobin gene from *Perosponia andersonii* (Landsman et al., Mol. Gen. Genet. 214 : 68-73 (1988)) or a mixture thereof.

A last aspect of the present invention is related to a transgenic plant tissue such as fruit, stem, root, tuber, seed of the transgenic plant according to the invention or a reproducible structure (preferably selected from the group consisting of calluses, buds or embryos) obtained from the transgenic plant or the cell according to the invention.

The techniques of plant transformation, tissue culture and regeneration used in the method according to the invention are the ones well known by the person skilled in the art. Such techniques are preferably the ones described in the International Patent Applications WO95/10178 or WO91/13159 corresponding to the European Patent Application EP-B-0517833, which are incorporated herein by reference. These techniques are preferably used for the preparation of transgenic sugar beets according to the invention.

### REFERENCES

1. Richards K.E. & Tamada T., *Annu. Rev. Phytopathol.* **30**, pp. 291-313 (1992)
2. Gilmer D. et al., *Virology* **189**, pp. 40-47 (1992)
3. Bouzoubaa S. et al., *J. Gen. Virol.* 68, pp. 615-626 (1987)
4. Herzog E. et al., *J. Gen. Virol.* **18**, pp. 3147-3155 (1994)
5. Scott K. P. et al., *J. Gen. Virol.* **75**, pp. 3561-3568 (1994)
6. Koonin E.V. & Dolja V.V., *Crit. Rev. Biochem. and Mol. Biol. 28,* pp. 375-430 (1993)
7. Schmitt C. et al., *Proc. Natl. Acad. Sci. USA.* **89**, pp. 5715-5719 (1992)
8. Morozov S.Y. et al., *J. Gen. Virol.* **72**, pp. 2039-2042 (1991)
22. Pelletier J. & Sonenberg N, *Nature* **334**, pp. 320-325 (1988)
23. Tamada T. & Baba T., *Annals of the Phytopathological Society of Japan* 39, pp. 325-332 (1973)
24. Kuszala M.& Putz C., *Annals of Phytopathology* **9,** pp. 435-446 (1977)
25. Tamada T., *CMI/AAB Description of Plants Viruses* **44**, (1975)
26. Asher M.J.C., *Rhizomania* In *The sugar beet crop,* ed. D.A. Cooke and R.K. Scott, Chapman & Hall, London, pp. 312-338 (1993)
27. Richard-Molard M., *Rhizomanie* In *Institut français de la betterave industrielle. Compte-rendu des travaux effectués en* 1994, ITB, Paris pp. 225-229 (1995)
28. Henry C.M. et al, *Plant Pathology* **41**, pp. 483-489 (1992)
29. Grassi G. et al., *Phytopath. Medit.* **28**, pp. 131-139 (1989)
30. Merdinoglu D. et al., *Acad. Agric. Fr.* **79**, n° 6, pp. 85-98 (1993)
31. Scolten O.E. et al., *Archives of Virology* **136**, pp. 349-361 (1994)
32. Büttenr G. & Bürcky K., *Proceedings of the First Symposium of the International Working Group on Plant Viruses with Fungal* Vectors, Braunschweig Germany, August 21-24 (1990)
33. Whitney E.D., *Plant Disease* **73,** pp. 287-289 (1989)
34. Powell A.P. et al., *Science* **232**, pp. 738-743 (1986)
35. Fritchen J.H. & Beachy R.N., *Ann. Rev. Microbiol.* **47**, pp. 739-763 (1993)
36. Wilson T.M.A., *Proc. Natl. Acad. Sci. USA* **90**, pp. 3134-3141 (1993)
37. Gonsalves D. & Slightom J.L., *Seminars in Virology* **4**, pp. 397-405 (1993)
38. D'Halluin K. et al., *Biotechnology* **10**, pp. 309-314 (1992)
39. Kallerhof J. et al., *Plant Cell Reports* **9**, pp. 224-228 (1990)
40. Ehlers U. et al., *Theoretical and Applied Genetic* **81**, pp. 777-782 (1991)
41. Kraus J. et al., *Field performance of transgenic sugar beet plants expresing BNYW coat protein plants, Fourth International Congress of Plant Molecular Biology, Int. Soc. for Plant Molecular Biology,* Amsterdam (1994)
42. Maiss E. et al., *Proceedings of the Third International Symposium on the Biosafety Results of Field Tests of Genetically Modified Plants and Microorganisms,* Monterey, pp. 129-139 (1994)
43. Norris et al., *Plant Molecular Biology* **21**, pp. 895-906 (1993)
44. Solovyev et al., *Virology* **219**, pp. 9-18 (1996)
45. Seppänen P. et al., *J. of General Virology* **78**, pp. 1241-1246 (1997)
46. Beck et al., *Proc. Natl. Acad. Sci. USA* **91**, pp. 10310-10314 (1994)
47. Cunningham et Wells, *Sciences* **244**, pp. 1081-1085 (1989)

## Claims

1. Method for inducing resistance to a group I virus comprising a TGB2 sequence into a plant cell or a plant, comprising the following steps:
- preparing a nucleotide construct comprising a nucleotide sequence corresponding to at least 70% of the nucleotide sequence of TGB2 of said virus or its complementary cDNA, being operably linked to one or more regulatory sequence(s) active in a plant,
- transforming a plant cell with the nucleotide construct, and possibly
- regenerating a transgenic plant from the transformed plant cell.

2. Method according to the claim 1, characterized in that the nucleotide sequence of the nucleotide construct corresponds to at least 80%, preferably at least 90%, of the nucleotide sequence of TGB2 of said virus or its complementary cDNA.

3. Method according to the claim 1 or 2, characterized in that the group I virus is selected from the group consisting of hordéiviruses, benyviruses, pecluviruses and pomoviruses, preferably selected from the group consisting of the beet necrotic yellow vein virus, the barley stripe mosaic virus, the potato mop top virus, the peanut clump virus and the beet soil-borne virus.

4. Method according to any of the preceding claims, characterized in that the plant cell is a stomatal cell.

5. Method according to any of the preceding claims, characterized in that the plant is selected from the group consisting of sugar beet, potato, barley or peanut.

6. Method according to claim 1 or 2, characterized in that the virus is BNYVV, the nucleotide sequence of TGB2 of said virus is comprised between the nucleotide 3287 and 3643 of the 5' strand of genomic or subgenomic RNA 2 of the BNYVV and the plant is a beet, preferably a sugar beet *(Beta vulgaris).*

7. Method according to any of the preceding claims, characterized in that the regulatory sequence comprises a promoter sequence or a terminator sequence active in a plant.

8. Method according to claim 7 characterized in that the promoter sequence is a constitutive or a foreigner promoter sequence.

9. Method according to the preceding claim 7, characterized in that the promoter sequence is selected from the group consisting of 35S Cauliflower Mosaic Virus promoter, and/or the polyubiquitin Arabidopsis thaliana promoter.

10. Method according to any of the claim 7 to 9, characterized in that the promoter sequence is a promoter which is capable of being active mainly into the root tissues of plants, such as the par promoter of the haemoglobin gene from Perosponia andersonii.

11. Transgenic plant resistant to a group I virus comprising a nucleotide construct having a nucleotide sequence corresponding to at least 70% of the nucleotide sequence of TGB2 of said virus or its corresponding cDNA, being operably linked to one or more regulatory sequence(s) active in a plant.

12. Transgenic plant according to the claim 11, characterized in that the nucleotide construct has a nucleotide sequence corresponding to at least 80%, preferably at least 90%, of the nucleotide sequence of TGB2 of said virus or its complementary cDNA.

13. Transgenic plant according to the claim 11 or 12, characterized in that the virus is selected from the group consisting of hordéiviruses, benyviruses, pecluviruses and pomoviruses, preferably selected from the group consisting of the beet necrotic yellow vein virus, the barley stripe mosaic virus, the potato mop top virus, the peanut clump virus and the beet soil-borne virus.

14. Transgenic plant according to the claims 11 to 13 being a plant selected from the group consisting of sugar beet, potato, barley or peanut.

15. Transgenic plant according to the claims 11 or 12, characterized in that the transgenic plant being a beet, preferably a sugar beet *(Beta vulgaris)* the virus is BNYVV and the nucleotide sequence of TGB2 of said virus is comprised between the nucleotides 3287 and 3643 of the 5' strand of genomic or subgenomic RNA 2 of BNYVV or its corresponding cDNA.

16. Transgenic plant according to any of the preceding claims 11 to 15, characterized in that the regulatory sequence comprises a promoter sequence and a terminator sequence active in a plant.

17. Transgenic plant according to any of the preceding claims 11 to 16, characterized in that the regulatory sequence(s) comprise a promoter sequence which is a constitutive or a foreigner promoter sequence.

18. Transgenic plant according to the claim 17, characterized in that promoter sequence is selected from the group consisting of 35S Cauliflower Mosaic Virus promoter, and/or the polyubiquitin *Arabidopsis thaliana* promoter.

19. Transgenic plant according to claim 17 or 18 characterized in that the promoter sequence is a promoter which is capable of being active mainly into root tissues, such as the *par* promoter of the haemoglobin gene from *Perosponia andersonii.*

20. Transgenic plant tissue selected from the group consisting of fruit, stem, root, tuber, seed of a plant according to any of the preceding claims 11 to 19.

21. Transgenic plant according to any one of the claims 11 to 19, characterised in that it further carries natural tolerance to Group I viruses.

22. Transgenic plant according to any one of the claims 11 to 19 and 21, characterised in that it further comprises a pesticide, herbicide or fungicide resistance, preferably a resistance selected from the group consisting of nematode resistance, glyphosate resistance, glufosomate resistance and/or acetochloride resistance.


### Annex to the application documents - subsequently filed sequences listing

SEQUENCE LISTING

(1) GENERAL INFORMATION:

(i) APPLICANT:
(A) NAME: CENTRE NATIONAL DE LA RECHERCHE SCIENTIFIQUE
(B) STREET: 3, Rue Michel-Ange
(C) CITY: PARIS CEDEX 16
(E) COUNTRY: FRANCE
(F) POSTAL CODE (ZIP): 75794

(ii) TITLE OF INVENTION: METHOD FOR INDUCING VIRAL RESISTANCE INTO A
PLANT

(iii) NUMBER OF SEQUENCES: 4

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.30 (EPO)

(2) INFORMATION FOR SEQ ID NO: 1:

(i) SEQUENCE CHARACTERISTICS:
(A) LENGTH: 357 base pairs
(B) TYPE: nucleic acid
(C) STRANDEDNESS: single
(D) TOPOLOGY: linear

(ii) MOLECULE TYPE: DNA (genomic)

(ix) FEATURE:
(A) NAME/KEY: CDS
(B) LOCATION:1..357

(xi) SEQUENCE DESCRIPTION: SEQ ID NO: 1:

ATG TCT AGG GAA ATA ACC GCT CGA CCC AAT AAG AAT GTG CCT ATT GTT 48
Met Ser Arg Glu Ile Thr Ala Arg Pro Asn Lys Asn Val Pro Ile Val
1               5                   10                  15

GTT GGT GTT TGT GTT GTG GCT TTC TTT GTA TTG CTG GCG TTC ATG CAG 96
Val Gly Val Cys Val Val Ala Phe Phe Val Leu Leu Ala Phe Met Gln
            20                  25                  30

CAA AAA CAT AAG ACA CAT TCT GGG GGT GAT TAC GGA GTC CCA ACA TTT 144
Gln Lys His Lys Thr His Ser Gly Gly Asp Tyr Gly Val Pro Thr Phe
        35                  40                  45

TCT AAC GGT GGT ATA TAT AGA GAC GGT ACA AGA TCA GCT GAT TTT AAT 192
Ser Asn Gly Gly Ile Tyr Arg Asp Gly Thr Arg Ser Ala Asp Phe Asn
    50                  55                  60

AGT AAC AAT CAT CGT GCT TAC GGG TGC GGT GGG TCT GGG GGT AGC GTT 240
Ser Asn Asn His Arg Ala Tyr Gly Cys Gly Gly Ser Gly Gly Ser Val
65                  70                  75                  80

AGT AGT CGA GTT GGG CAG CAA CTT ATT GTG TTA GCT ATT GTT TCT GTG 288
Ser Ser Arg Val Gly Gln Gln Leu Ile Val Leu Ala Ile Val Ser Val
                85                  90                  95

TTA ATA GTG TCA CTA TTA CAA CGA TTA AGG TCT CCA CCA GAA CAC ATT 336
Leu Ile Val Ser Leu Leu Gln Arg Leu Arg Ser Pro Pro Glu His Ile
            100                 105                 110

TGT AAT GGT GCT TGT GGT TAA 357
Cys Asn Gly Ala Cys Gly *
        115

(2) INFORMATION FOR SEQ ID NO: 2:

(i) SEQUENCE CHARACTERISTICS:
(A) LENGTH: 119 amino acids
(B) TYPE: amino acid
(D) TOPOLOGY: linear

(ii) MOLECULE TYPE: protein
(xi) SEQUENCE DESCRIPTION: SEQ ID NO: 2:

Met Ser Arg Glu Ile Thr Ala Arg Pro Asn Lys Asn Val Pro Ile Val
1               5                   10                  15

Val Gly Val Cys Val Val Ala Phe Phe Val Leu Leu Ala Phe Met Gln
            20                  25                  30

Gln Lys His Lys Thr His Ser Gly Gly Asp Tyr Gly Val Pro Thr Phe
        35                  40                  45

Ser Asn Gly Gly Ile Tyr Arg Asp Gly Thr Arg Ser Ala Asp Phe Asn
    50                  55                  60

Ser Asn Asn His Arg Ala Tyr Gly Cys Gly Gly Ser Gly Gly Ser Val
65                  70                  75                  80

Ser Ser Arg Val Gly Gln Gln Leu Ile Val Leu Ala Ile Val Ser Val
                85                  90                  95

Leu Ile Val Ser Leu Leu Gln Arg Leu Arg Ser Pro Pro Glu His Ile
            100                 105                 110

Cys Asn Gly Ala Cys Gly *
        115

(2) INFORMATION FOR SEQ ID NO: 3:

(i) SEQUENCE CHARACTERISTICS:
(A) LENGTH: 357 base pairs
(B) TYPE: nucleic acid
(C) STRANDEDNESS: single
(D) TOPOLOGY: linear

(ii) MOLECULE TYPE: DNA (genomic)

(ix) FEATURE:
(A) NAME/KEY: CDS

(B) LOCATION:1..357

(xi) SEQUENCE DESCRIPTION: SEQ ID NO: 3:

ATG TCT AGG GAA ATA ACC GCT CGA CCC AAT AAG AAT GTG CCT ATT GTT 48
Met Ser Arg Glu Ile Thr Ala Arg Pro Asn Lys Asn Val Pro Ile Val
120 125 130 135

GTT GGT GTT TGT GTT GTG GCT TTC TTT GTA TTG CTG GCG TTC ATG CAG 96
Val Gly Val Cys Val Val Ala Phe Phe Val Leu Leu Ala Phe Met Gln
140 145 150

CAA GCA GCT GCG ACA CAT TCT GGG GGT GAT TAC GGA GTC CCA ACA TTT 144
Gln Ala Ala Ala Thr His Ser Gly Gly Asp Tyr Gly Val Pro Thr Phe
155 160 165

TCT AAC GGT GGT ATA TAT AGA GAC GGT ACA AGA TCA GCT GAT TTT AAT 192
Ser Asn Gly Gly Ile Tyr Arg Asp Gly Thr Arg Ser Ala Asp Phe Asn
170 175 180

AGT AAC AAT CAT CGT GCT TAC GGG TGC GGT GGG TCT GGG GGT AGC GTT 240
Ser Asn Asn His Arg Ala Tyr Gly Cys Gly Gly Ser Gly Gly Ser Val
185 190 195

AGT AGT CGA GTT GGG CAG CAA CTT ATT GTG TTA GCT ATT GTT TCT GTG 288
Ser Ser Arg Val Gly Gln Gln Leu Ile Val Leu Ala Ile Val Ser Val
200 205 210 215

TTA ATA GTG TCA CTA TTA CAA CGA TTA AGG TCT CCA CCA GAA CAC ATT 336
Leu Ile Val Ser Leu Leu Gln Arg Leu Arg Ser Pro Pro Glu His Ile
220 225 230

TGT AAT GGT GCT TGT GGT TAA 357
Cys Asn Gly Ala Cys Gly *
235

(2) INFORMATION FOR SEQ ID NO: 4:

(i) SEQUENCE CHARACTERISTICS:
(A) LENGTH: 119 amino acids
(B) TYPE: amino acid
(D) TOPOLOGY: linear

(ii) MOLECULE TYPE: protein
(xi) SEQUENCE DESCRIPTION: SEQ ID NO: 4:

Met Ser Arg Glu Ile Thr Ala Arg Pro Asn Lys Asn Val Pro Ile Val
1               5                   10                  15

Val Gly Val Cys Val Val Ala Phe Phe Val Leu Leu Ala Phe Met Gln
            20                  25                  30

Gln Ala Ala Ala Thr His Ser Gly Gly Asp Tyr Gly Val Pro Thr Phe
        35                  40                  45

Ser Asn Gly Gly Ile Tyr Arg Asp Gly Thr Arg Ser Ala Asp Phe Asn
    50                  55                  60

Ser Asn Asn His Arg Ala Tyr Gly Cys Gly Gly Ser Gly Gly Ser Val

65                  70                  75                  80

Ser Ser Arg Val Gly Gln Gln Leu Ile Val Leu Ala Ile Val Ser Val
                85                  90                  95

Leu Ile Val Ser Leu Leu Gln Arg Leu Arg Ser Pro Pro Glu His Ile
            100                 105                 110

Cys Asn Gly Ala Cys Gly  *
        115

SEQUENCE LISTING

<110> CENTRE NATIONAL DE LA RECHERCHE SCIENTIFIQUE

<120> METHOD FOR INDUCING VIRAL RESISTANCE INTO A PLANT

<130> P.SES.03/EP

<140> 99200773.2
<141> 1999-03-12

<160> 4

<170> PatentIn Ver. 2.1

<210> 1
<211> 357
<212> DNA
<213> Beet necrotic yellow vein virus

<220>
<221> CDS
<222> (1)..(354)

<400> 1
atg tct agg gaa ata acc gct cga ccc aat aag aat gtg cct att gtt 48
Met Ser Arg Glu Ile Thr Ala Arg Pro Asn Lys Asn Val Pro Ile Val
1               5                   10                  15

gtt ggt gtt tgt gtt gtg gct ttc ttt gta ttg ctg gcg ttc atg cag 96
Val Gly Val Cys Val Val Ala Phe Phe Val Leu Leu Ala Phe Met Gln
            20                  25                  30

caa aaa cat aag aca cat tct ggg ggt gat tac gga gtc cca aca ttt 144
Gln Lys His Lys Thr His Ser Gly Gly Asp Tyr Gly Val Pro Thr Phe
        35                  40                  45

tct aac ggt ggt ata tat aga gac ggt aca aga tca gct gat ttt aat 192
Ser Asn Gly Gly Ile Tyr Arg Asp Gly Thr Arg Ser Ala Asp Phe Asn
    50                  55                  60

agt aac aat cat cgt gct tac ggg tgc ggt ggg tct ggg ggt agc gtt 240
Ser Asn Asn His Arg Ala Tyr Gly Cys Gly Gly Ser Gly Gly Ser Val
65                  70                  75                  80

agt agt cga gtt ggg cag caa ctt att gtg tta gct att gtt tct gtg 288
Ser Ser Arg Val Gly Gln Gln Leu Ile Val Leu Ala Ile Val Ser Val
                85                  90                  95

tta ata gtg tca cta tta caa cga tta agg tct cca cca gaa cac att 336
Leu Ile Val Ser Leu Leu Gln Arg Leu Arg Ser Pro Pro Glu His Ile
            100                 105                 110

tgt aat ggt gct tgt ggt taa 357
Cys Asn Gly Ala Cys Gly
        115

<210> 2
<211> 118
<212> PRT

<213> Beet necrotic yellow vein virus

<400> 2
Met Ser Arg Glu Ile Thr Ala Arg Pro Asn Lys Asn Val Pro Ile Val
1               5                   10                  15

Val Gly Val Cys Val Val Ala Phe Phe Val Leu Leu Ala Phe Met Gln
            20                  25                  30

Gln Lys His Lys Thr His Ser Gly Gly Asp Tyr Gly Val Pro Thr Phe
        35                  40                  45

Ser Asn Gly Gly Ile Tyr Arg Asp Gly Thr Arg Ser Ala Asp Phe Asn
    50                  55                  60

Ser Asn Asn His Arg Ala Tyr Gly Cys Gly Gly Ser Gly Gly Ser Val
65                  70                  75                  80

Ser Ser Arg Val Gly Gln Gln Leu Ile Val Leu Ala Ile Val Ser Val
                85                  90                  95

Leu Ile Val Ser Leu Leu Gln Arg Leu Arg Ser Pro Pro Glu His Ile
            100                 105                 110

Cys Asn Gly Ala Cys Gly
        115

<210> 3
<211> 357
<212> DNA
<213> Beet necrotic yellow vein virus

<220>
<221> CDS
<222> (1)..(354)

<400> 3
atg tct agg gaa ata acc gct cga ccc aat aag aat gtg cct att gtt      48
Met Ser Arg Glu Ile Thr Ala Arg Pro Asn Lys Asn Val Pro Ile Val
1               5                   10                  15

gtt ggt gtt tgt gtt gtg gct ttc ttt gta ttg ctg gcg ttc atg cag      96
Val Gly Val Cys Val Val Ala Phe Phe Val Leu Leu Ala Phe Met Gln
            20                  25                  30

caa gca gct gcg aca cat tct ggg ggt gat tac gga gtc cca aca ttt      144
Gln Ala Ala Ala Thr His Ser Gly Gly Asp Tyr Gly Val Pro Thr Phe
        35                  40                  45

tct aac ggt ggt ata tat aga gac ggt aca aga tca gct gat ttt aat      192
Ser Asn Gly Gly Ile Tyr Arg Asp Gly Thr Arg Ser Ala Asp Phe Asn
    50                  55                  60

agt aac aat cat cgt gct tac ggg tgc ggt ggg tct ggg ggt agc gtt      240
Ser Asn Asn His Arg Ala Tyr Gly Cys Gly Gly Ser Gly Gly Ser Val
65                  70                  75                  80

agt agt cga gtt ggg cag caa ctt att gtg tta gct att gtt tct gtg      288
Ser Ser Arg Val Gly Gln Gln Leu Ile Val Leu Ala Ile Val Ser Val

                85                  90                  95

tta ata gtg tca cta tta caa cga tta agg tct cca cca gaa cac att 336
Leu Ile Val Ser Leu Leu Gln Arg Leu Arg Ser Pro Pro Glu His Ile
            100                 105                 110

tgt aat ggt gct tgt ggt taa 357
Cys Asn Gly Ala Cys Gly
        115

<210> 4
<211> 118
<212> PRT
<213> Beet necrotic yellow vein virus

<400> 4
Met Ser Arg Glu Ile Thr Ala Arg Pro Asn Lys Asn Val Pro Ile Val
1               5                   10                  15

Val Gly Val Cys Val Val Ala Phe Phe Val Leu Leu Ala Phe Met Gln
            20                  25                  30

Gln Ala Ala Ala Thr His Ser Gly Gly Asp Tyr Gly Val Pro Thr Phe
        35                  40                  45

Ser Asn Gly Gly Ile Tyr Arg Asp Gly Thr Arg Ser Ala Asp Phe Asn
    50                  55                  60

Ser Asn Asn His Arg Ala Tyr Gly Cys Gly Gly Ser Gly Gly Ser Val
65                  70                  75                  80

Ser Ser Arg Val Gly Gln Gln Leu Ile Val Leu Ala Ile Val Ser Val
                85                  90                  95

Leu Ile Val Ser Leu Leu Gln Arg Leu Arg Ser Pro Pro Glu His Ile
            100                 105                 110

Cys Asn Gly Ala Cys Gly
        115